# EUROPEAN PATENT APPLICATION

(11) **EP 4 389 127 A1**
(43) Date of publication of application: **26.06.2024**
(21) Application number: 22857799.5
(22) Date of filing: 16.08.2022
(51) Int. Cl.: A61K 31/497, A61P 9/10, A61P 43/00, A61P 25/00, A61P 27/02

(54) **USE OF AND METHOD FOR TELAPREVIR IN PREPARATION OF DRUG AND CYTOPROTECTIVE DRUG FOR TREATING ISCHEMIA/REPERFUSION INJURY**

(30) Priority: 17.08.2021 CN 202110943155
(71) Applicant: The Third Xiangya Hospital of Central South University, Changsha, Hunan 410013 (CN)
(72) Inventor: LUO, Xiuju, Changsha, Hunan 410013 (CN); PENG, Jun, Changsha, Hunan 410013 (CN); ZHANG, Yiyue, Changsha, Hunan 410013 (CN); LI, Mingrui, Changsha, Hunan 410013 (CN)
(74) Representative: karo IP
(86) International application number: PCT/CN2022/112771
(87) International publication number: WO 2023/020488

(57) **Abstract**

A use of and method for telaprevir in the preparation of a drug and a cytoprotective drug for treating ischemia/reperfusion injury. Telaprevir is used for the preparation of a drug for treating ischemia/reperfusion injury, in particular for treating myocardial cells and nerve cell damage, said drug having in particular a good protective effect on heart and cerebral ischemia/reperfusion (myocardial infarction and ischemic stroke), and can significantly alleviate heart and cerebral ischemia/reperfusion injury.

## Description

### Field of the Invention

The present invention relates to a use of telaprevir in preparation of a drug for treating an ischemia/reperfusion injury and a cytoprotective drug, and relates to a method thereof, belonging to the field of biomedicine. The present invention provides a novel use and administration method of telaprevir for protecting heart and brain ischemia/reperfusion (especially myocardial infarction and ischemic stroke) and alleviating an ischemia/reperfusion injury, and a use of a cytoprotective drug for protecting organs, tissues, or cells against cell injuries and dysfunctions, and expanding a scope of indications for telaprevir. In addition, the present invention further covers a use of telaprevir in preparation of protective drugs against myocardial cell injuries and nerve cell injuries.

### Background of the Invention

Myocardial infarction is mainly caused by heart ischemia. If no timely reperfusion or collateral circulation is implemented, myocardial cells will be irreversible injuried and be replaced by fibrous scar tissues which has almost no contractile function. Cerebral infarction, also known as ischemic stroke, is a local brain tissue blood supply disorder caused by many reasons, leading to cerebral ischemia, hypoxic necrosis, and neurological deficit. The ischemic stroke is a common and frequently occurring disease that seriously endangers human health. It has become the first cause of disability and the third cause of death in the world. The incidence rate of the ischemic stroke accounts for 70-80% of cerebrovascular diseases.

One of the main measures for infarction treatment is to restore perfusion blood supply to infarcted tissues as soon as possible, but reperfusion treatment often accompanies some tissue injuries, and causes pathological and physiological changes. The injury caused by ischemia and reperfusion is referred to as "ischemia/reperfusion (I/R) injury". The I/R injury involves multiple mechanisms such as oxidative stress, calcium overload, energy metabolism disorder, and inflammatory response, leads to multiple modes of cell death such as apoptosis and necrosis. Research has shown that inhibiting apoptosis and/or necrosis can inhibit myocardial and nerve cell death caused by ischemia/reperfusion, alleviate the degrees of heart or brain ischemia/reperfusion injury, and narrow the infarct range.

Alzheimer's disease (AD) is a common central neurodegenerative disease, occurs mainly in the elderly population, and is also known as senile dementia. Clinically, it manifests as a decline in learning and memory, a decline in cognitive function, visual spatial disorders, personality and behavioral changes, and even complete dementia in severe cases. The Alzheimer's disease has main pathological features of precipitation and accumulation of a β-amyloid protein in cell matrices to form senile plaques, excessive phosphorylation of a tau protein to form neurofibrillary tangles, neuronal degeneration and loss, and its nerve cell injury also relates to oxidative stress, inflammatory response, mitochondrial dysfunction, and other mechanisms. AD lacks effective therapeutic drugs. Existing drugs can alleviate only some symptoms and delay courses of diseases, but cannot cure AD, and have obvious side effects. With the aggravation of population aging, the incidence rate of AD will increase year by year. It is of great significance to seek effective drugs to treat AD.

Telaprevir is a hepatitis C virus (HCV) NS3/4A serine protease inhibitor that has antiviral effects by inhibiting HCV replication, but whether it has an anti-ischemia/reperfusion injury or anti-AD effect has not yet been reported.

In the present invention, unless otherwise specified, "telaprevir" should be understood as "a compound of telaprevir and any stereoisomer thereof or one of their semi-synthetic derivatives or one of their salts (salts of the compound or salts of the semi-synthetic derivatives) or one of their esters (esters of the compound or esters of the semi-synthetic derivatives) or one of their ester salts (salts of the esters of the compound or salts of the esters of the semi-synthetic derivatives)" or "deuteratedtelaprevir"; or a mixture of a telaprevir compound and a single component of any stereoisomer thereof or any proportion of any stereoisomer.

### Summary of the Invention

In response to the shortcomings of existing technologies, a first objective of the present invention is to provide a use of telaprevir in preparation of a drug for treating or preventing an ischemia/reperfusion injury or an Alzheimer's disease and a cytoprotective drug; a second objective of the present invention is to provide a use of telaprevir in preparation of a cytoprotective drug; a third objective of the present invention is to provide a use of telaprevir in treatment or prevention of an ischemia/reperfusion injury or an Alzheimer's disease; and a fourth objective of the present invention is to provide a method for treating or preventing an ischemia/reperfusion injury or an Alzheimer's disease with telaprevir.

The inventor found that telaprevir has an anti-hypoxia/reoxygenation or NMDA induced myocardial and/or nerve cell injury effect, can reduce myocardial and nerve cell death caused by myocardial and cerebral ischemia, significantly reduce the infarct volume (area) of heart and brain ischemia, reduce the activity of a serum creatine kinase, improve neurological functions, and reduce myocardial and nerve cell death, and has an effect of protecting nerve cells and myocardial cells, and its effect of alleviating heart and brainischemia/reperfusion injury is superior to that of current commonly used cardiovascular and cerebrovascular drugs. The inventor found that the telaprevir has an effect of protecting nerve cells and can significantly improve learning and memory ability and cognitive function in an Alzheimer's disease.

Optionally, a structural formula of the telaprevir is as shown in formula I, with a molecular formula C₃₆H₅₃N₇O₆.

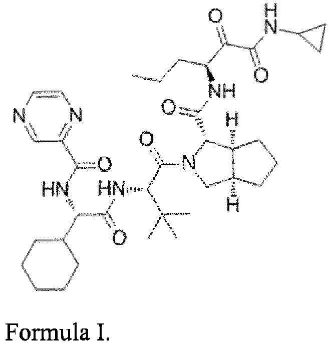

In order to solve the above technical problems, technical solutions of the present invention are as follows:
A use of telaprevir in preparation of a drug for treating or preventing an ischemia/reperfusion injury.

Further, the ischemia/reperfusion injury includes one or more of a myocardial ischemia/reperfusion injury, a cerebral ischemia/reperfusion injury, and a liver and kidney ischemia/reperfusion injury.

Further, the myocardial ischemia/reperfusion injury includes an ischemic heart disease.

Further, the cerebral ischemia/reperfusion injury includes an ischemic stroke.

Further, the ischemia/reperfusion injury includes myocardial infarction.

Further, the drug is administered by one or more of intramuscular injection, subcutaneous injection, intravenous injection, oral administration, sublingual administration, intralesional or intracerebral or implanted delivery, and spray administration, and preferably intramuscular, subcutaneous or intravenous injection. Further, the drug is administered by intramuscular, subcutaneous, or intravenous injection.

Further, the drug may be prepared into any pharmaceutically acceptable dosage form. Further, the dosage form includes one of injection, capsules, tablets, granules, suspension, emulsion, spray, powder, oral liquid, dropping pills, and liposome, where a preferred dosage form is injection.

Optionally, the telaprevir is a pharmaceutically acceptable salt thereof, the pharmaceutically acceptable salt is a pharmaceutically commonly used salt, and further, the salt is selected from one or more of acetate, hydrochloride, hydrobromide, nitrate, sulfate, phosphate, benzoate, fumarate, maleate, succinate, tartarate, citrate, oxalate, glyoxylate, aspartate, tartrate, 2,5-dihydroxybenzoate, methanesulfonate, ethanesulfonate, benzene sulfonate, lauryl sulfonate, hydroquinone sulfonate, and p-toluenesulfonate.

A use of telaprevir in preparation of a cytoprotective drug.

Further, the cytoprotective drug is a drug that has the effects of preventing, inhibiting, or treating injury, degeneration, or dysfunction of tissues, organs, and cells. Optionally, the cytoprotective drug includes drugs used in organs such as the nervous system, brain, heart, and eyes or cells against cell death or a process causing cell death, such as drugs used for treating diseases caused by necrosis and/or pathological apoptosis and/or necroptosis and/or ferroptosis and/or cell pyroptosis and/or autophagy.

Further, the cytoprotective drug is a drug for preventing, inhibiting, or treating cardiovascular, neurological, or ophthalmic diseases.

Further, the organs include one or more of the brain, lungs, heart, blood vessels, kidneys, pancreas, skin, eyes, and corneas.

Optionally, the cytoprotective drug is a drug used for treating or preventing a myocardialcell injury or a nerve cell injury.

Further, the cells include one or more of myocardial cells and nerve cells.

Preferably, the organs in the present invention include the nervous system, brain, and heart. One of the objectives of the present invention is to provide a novel use of telaprevir (a single compound or a pharmaceutical composition containing the compound) for preventing and/or treating cell death or a process causing cell death, such as cell death caused by necrosis and/or pathological apoptosis and/or necrotic apoptosis and/or ferroptosis and/or pyroptosis and/or autophagy.

Further, the telaprevir can protect, prevent, and/or treat cell death or a process causing cell death, including inhibiting nerve cell injury and death, such as treating and preventing neurological diseases, glaucoma, retinitis pigmentosa, corneal network malnutrition, age-related macular degeneration (AMD), wet or dry AMD related photoreceptor degeneration, other retinal degeneration, optic neuropathy, optic neuritis, optic nerve drusen, stroke, Alzheimer's disease, Parkinson's disease, Huntington's disease, Parkinson's plus syndrome, local ischemia, amyotrophic lateral sclerosis (ALS), intracranial hemorrhage, cerebral hemorrhage, trigeminal neuralgia, glossopharyngeal neuralgia, myasthenia gravis, muscular atrophy, progressive muscular atrophy, Bell's palsy, progressive bulbar paralysis, spinal muscular atrophy, primary lateral sclerosis (PLS), pseudobulbar palsy, invertebrate disc syndrome, cervical spondylosis, hereditary muscle atrophy, plexus disorder, thoracic outlet disruption syndrome, porphyria, peripheral neuropathy, multiple system atrophy, cortical basal ganglia degeneration, progressive supranuclear palsy, dementia with lewy bodies, demyelinating disease, frontotemporal lobe dementia, Guillain-Barre syndrome, multiple sclerosis, Creutzfeldt-Jakob disease, Charcot-Marie-Tooth disease, prion disease, fatal familial insomnia (FFI), Gerstmann-Straussler-Scheinker syndrome (GSS), bovine spongiform encephalopathy, epilepsy, Pick's disease, AIDS dementia syndrome, nerve injury caused by exposure to toxic compounds in a group consisting of industrial solvents, heavy metals, drugs, and chemotherapy agents, and nervous system injury caused by mechanical, physical, or chemical trauma.

Further, the telaprevir can protect, prevent, and/or treat cell death or a process causing cell death, including inhibiting myocardial cell injury and death, such as treating and preventing cardiovascular diseases, heart ischemia and/or vascular ischemia, myocardial infarction, ischemic heart disease, myocardial remodeling, chronic or acute heart failure, hypertrophic cardiomyopathy, and cardiac side effects caused by drug therapy (especially anti-cancer drugs).

A use of telaprevir in preparation of a cytoprotective drug for myocardial ischemia/reperfusion injury and/or cerebral ischemia/reperfusion injury.

Further, the cardiovascular diseases include one or more of cardiac ischemia and/or vascular ischemia, myocardial infarction, ischemic heart disease, myocardial remodeling, chronic or acute heart failure, hypertrophic cardiomyopathy, cardiac side effects caused by drug therapy, cerebral ischemia/reperfusion injury, liver ischemia/reperfusion injury, and kidney ischemia/reperfusion injury; and the neurological diseases include one or more of glaucoma, retinitis pigmentosa, corneal network malnutrition, age-related macular degeneration, wet or dry AMD related photoreceptor degeneration, optic neuropathy, optic neuritis, optic nerve drusen, stroke, Alzheimer's disease, Parkinson's disease, Huntington's disease, Parkinson's plus syndrome, local ischemia, amyotrophic lateral sclerosis, intracranial hemorrhage, cerebral hemorrhage, trigeminal neuralgia, glossopharyngeal neuralgia, myasthenia gravis, muscular atrophy, progressive muscular atrophy, Bell's palsy, progressive bulbar paralysis, spinal muscular atrophy, primary lateral sclerosis, pseudobulbar palsy, invertebrate disc syndrome, cervical spondylosis, hereditary muscle atrophy, plexus disorder, thoracic outlet disruption syndrome, porphyria, peripheral neuropathy, multiple system atrophy, cortical basal ganglia degeneration, progressive supranuclear palsy, dementia with lewy bodies, demyelinating disease, frontotemporal lobe dementia, Guillain-Barre syndrome, multiple sclerosis, Creutzfeldt-Jakob disease, Charcot-Marie-Tooth disease, prion disease, fatal familial insomnia, Gerstmann-Straussler-Scheinker syndrome, bovine spongiform encephalopathy, epilepsy, Pick's disease, AIDS dementia syndrome, nerve injury caused by exposure to toxic compounds in a group consisting of industrial solvents, heavy metals, drugs, and chemotherapy agents, and nervous system injury caused by mechanical, physical, or chemical trauma.

A use of telaprevir in treatment or prevention of an ischemia/reperfusion injury. Further, the ischemia/reperfusion injury is caused by one or more of a myocardial ischemia/reperfusion injury, a cerebral ischemia/reperfusion injury, a liver ischemia/reperfusion injury, and a kidney ischemia/reperfusion injury.

Further, the myocardial ischemia/reperfusion injury is caused by an ischemic heart disease.

Further, the cerebral ischemia/reperfusion injury is caused by an ischemic stroke.

A method for treating or preventing an ischemia/reperfusion injury with telaprevir, an effective dose of telaprevir is administrated to patients or in vitro tissues and organs. Further, the ischemia/reperfusion injury is caused by one or more of a myocardial ischemia/reperfusion injury, a cerebral ischemia/reperfusion injury, a liver ischemia/reperfusion injury, and a kidney ischemia/reperfusion injury.

Further, the myocardial ischemia/reperfusion injury is caused by an ischemic heart disease.

Further, the cerebral ischemia/reperfusion injury is caused by an ischemic stroke.

A use of telaprevir in protection of cells.

Further, the cells include one or more of myocardial cells and nerve cells.

A method for protecting cells with telaprevir, an effective dose of telaprevir is administrated to patients or in vitro tissues and organs.

Optionally, the in vitro tissues and organs include one or more of the heart, liver, kidneys, and brain.

Optionally, the in vitro tissues and organs come from human bodies or non-human animals.

Further, the cells include one or more of myocardial cells and nerve cells.

A use of telaprevir in preparation of a drug for treating or preventing an Alzheimer's disease.

A use of telaprevir in preparation of a drug for improving a cognitive function in an Alzheimer's disease.

A use of telaprevir in treatment or prevention of an Alzheimer's disease.

A use of telaprevir in improvement on a cognitive function in an Alzheimer's disease. A method for treating or preventing an Alzheimer's disease with telaprevir, an effective dose of telaprevir is administrated to patients.

A method for improving a cognitive function in an Alzheimer's disease with telaprevir, an effective dose of telaprevir is administrated to patients.

A use of the drug mentioned above in preparation of an anti-cancer drug for preventing or treating cardiovascular toxicity, the anti-cancer drug includes but is not limited to anthracycline antibiotics, tyrosine kinase inhibitors, fluorouracils, VEGF signaling pathway inhibitors, immune checkpoint inhibitors, platinum anti-tumor drugs, and other anti-tumor drugs.

Further, the anticancer drug includes anthracycline antibiotics, such as adriamycin, epirubicin, pirarubicin, aclarubicin, idarubicin, daunorubicin, and mitoxantrone; tyrosine kinase inhibitors, such as nilotinib, sunitinib, lapatinib, regorafenib, ponatinib, and dasatinib; fluorouracils, such as fluorouracil, capecitabine, tegafurgimeraciloteracil potassium, and tegafur; VEGF signaling pathway inhibitors, such as bevacizumab; immune checkpoint inhibitors, such as trastuzumab; platinum anti-tumor drugs, such as cis-platinum; and other anti-tumor drugs, such as paclitaxel and cyclophosphamide. The inventor unexpectedly discovered that telaprevir has an anti-hypoxia/reoxygenation or NMDA induced myocardial and/or nerve cell injury effect, can reduce myocardial and nerve cell death caused by myocardial ischemia/reperfusion and cerebral ischemia/reperfusion, significantly reduce the infarct volume (area) of heart and brain ischemia, reduce the activity of a serum creatine kinase, and improve neurological functions, and has an effect of protecting myocardial and nerve cells. The drug of the present invention has a better effect of alleviating a heart and brain ischemia/reperfusion injury than commonly used cerebrovascular drugs, may be used for treating myocardial infarction and ischemic stroke, and may be further used for the protection of in vitro organs such as the heart and brain, with good prospects of development and application. The inventor found that the telaprevir has an effect of protecting nerve cells and can significantly improve learning and memory ability and cognitive function in an Alzheimer's disease.

The present invention expands indications of telaprevir and can be applied to ischemic stroke, myocardial infarction, cell protection, and Alzheimer's disease.

The present invention discloses a use of telaprevir in preparation of a drug for treating an ischemia/reperfusion injury and a cytoprotective drug for the first time. It is unexpected that the telaprevir can significantly reduce the volume (area) of myocardial infarction and cerebral infarction, reduce the activity of a serum creatine kinase, improve neurological functions, and reduce myocardial cell and nerve cell death, and has a protective effect on nerve cells and myocardial cells. This is unrelated to the known uses of telaprevir, has not been enlightened by other existing compounds, and has prominent substantive characteristics and significant progress in treatment of an ischemia/reperfusion injury and cell protection. The present invention found that the telaprevir has a protective effect on nerve cells and can significantly improve learning and memory ability and cognitive function in an Alzheimer's disease.

### Brief Description of the Drawings:

FIG. 1 is a diagram illustrating effects of telaprevir on myocardial infarct area and serum creatine kinase activity of mice in Example 1, where the telaprevir was administrated after 30 minutes of ischemia. A-measurement on the TTC staining and infarct area of myocardial tissues in mice after administration of telaprevir, and B-serum creatine kinase activity after administration of telaprevir.
FIG. 2 is a diagram illustrating effects of telaprevir on cerebral infarct volume and neurological function of rats in Example 2, where the telaprevir was administrated after 2 hours of ischemia and 1 hour of reperfusion. A-measurement on the TTC staining and infarct volume of rat brain tissues, and B-scores on the neurological function of rats.
FIG. 3 is a diagram illustrating effects of telaprevir on cerebral infarct volume and neurological function of mice in Example 3, where the telaprevir was administrated after 1 hour of ischemia and 1 hour of reperfusion. A-measurement on the TTC staining and infarct volume of mouse brain tissues, and B-scores on the neurological function of mice.
FIG. 4 is a diagram illustrating effects of telaprevir on hypoxic/reoxygenated myocardial cells in Example 4.
FIG. 5 is a diagram illustrating effects of telaprevir on nerve cells treated by hypoxia/reoxygenation and NMDA in Example 5.
FIG. 6 is a diagram illustrating effects of telaprevir on the spatial learning and memory ability of Alzheimer's disease model mice in Example 6. A-time that mice found an original platform (incubation period), and B-times that mice cross the platform.
FIG. 7 is a diagram illustrating effects of telaprevir on the non-spatial learning and memory ability of Alzheimer's disease model mice in Example 6.

### Detailed Description of the Embodiments:

The present invention will be explained in detail below in conjunction with examples. A use of telaprevir in preparation of a drug for treating an ischemia/reperfusion injury and a drug with a cellular protective effect.

### Materials and methods:

To demonstrate the effects of telaprevir in anti-ischemia/reperfusion injury and cell protection, the applicant used myocardial ischemia/reperfusion injury mouse models and ischemic stroke rat models, administrated animals with telaprevir at different time after ischemia/reperfusion, animal death after 24 hours of reperfusion, detected a myocardial cell injury by TTC staining and serum creatine kinase activity, and detected a nerve cell injury by neurobiological scores and TTC staining.

To demonstrate the protective effect of telaprevir on cells, the applicant used hypoxia/reoxygenation injury models and NMDA treatment models, administrated animals with telaprevir, and detected cell viability and cell injury by MTS and LDH, respectively.

To demonstrate the protective effect of telaprevir on nerve cells, the applicant administered Alzheimer's disease mouse models with telaprevir, and detected a nerve cell injury and a learning and memory ability and cognitive function of mice by Morris water maze and new/old object recognition experiments.

Implementing drug used in each example: telaprevir (a compound of telaprevir, a structural formula of which is shown in formula I), purchased from a reagent manufacturers.

### Example 1

Animal experiment: Protective effect of telaprevir on myocardial ischemia/reperfusion injury mouse models.

Experimental animals: 7-week-old male C57BL/6J mice. The experimental animals were raised for one week in an environment with a temperature of 25°C, a relative humidity of 60%, free drinking water, and timed and quantified feeding, and then administered according to the requirements of each experimental group.

Method for building a myocardial ischemia/reperfusion mouse model: After an 8-week-old male C57BL/6J mouse was anesthetized by intraperitoneal injection with 0.3% pentobarbital sodium (20 mL/kg), a trachea cannula was inserted from the mouth and secured with a pressure-sensitive adhesive. Parameters of a ventilator were set to a tidal volume of 3.2 mL/kg and a frequency of 110 times per minute. The chest was opened, left anterior descending (LAD) branches were ligated with 8-0 sutures, myocardial infarction can be determined when the cardiac apex turned white, and the ligation was maintained for 1 hour. After 1 hour of ischemia, LAD suture knots were opened to restore blood flow to the heart, the chest was sutured layer by layer, the ventilator was removed, and the mouse can resume autonomous breathing. After 24 hours of cardiac reperfusion, the mouse was anesthetized with 0.3% pentobarbital sodium (20 mL/kg), the chest was opened, the LAD branches were ligated in situ again. The abdominal cavity was opened and injected with 0.2 mL of 2% Evans Blue solution through an inferior vena cava. When the lower lip of the mouse turned blue, blood was collected from the cardiac apex and the heart was taken out. After the heart was frozen at -20°C for 1 hour, the heart was cut into 1 mm thick slices, and 1% TTC staining solution (prepared with PBS) was added to incubate the slices at 37°C for 15 minutes. After staining, the staining solution was removed, the heart slices were washed with PBS once, immobilized with 4% paraformaldehyde for 24 hours, and then taken out, the staining situation was observed, photos were taken, and an ischemic area and an infarct area were measured by ImageJ software.

A blue area of the heart slice represented normal tissues; the area of the heart slice, except for the blue area, was an ischemic area (also known as an area of risk, AOR); a white area of the heart slice was an area of infarction (AOI), a percentage of infarct area (a ratio of AOI to AOR) of each slice was calculated, and a ratio of areas of infarction of a drug group and a model group was calculated.

Experimental groups: the experimental animals were randomly divided into 4 groups, namely:
Sham group: a surgery was performed on the heart of the mouse without vascular ligation;
Myocardial ischemia/reperfusion group (I/R group): the left anterior descending coronary arteries were ligated for 1 hour, and sutures were cut, followed by reperfusion for 24 hours;
Telaprevir + myocardial ischemia/reperfusion group (Telaprevir + I/R): administered with telaprevir (60 mg/kg) after 30 minutes of ischemia.
Vehicle + myocardial ischemia/reperfusion group (Vehicle + I/R): administered with a vehicle after 30 minutes of ischemia.
Blood and myocardial tissues were collected and relevant indicators were measured: myocardial infarct area in mice was measured and serum creatine kinase activity (CK activity) was detected.

### Detection on serum creatine kinase (CK) activity

After the ischemia/reperfusion surgery, about 150 µL of whole blood was collected from the orbit of each mouse and centrifuged at 3000 rpm and 4°C for 10 minutes, and then the supernatant was taken and stored at -40°C. The serum CK activity was measured by the following steps according to the instructions of a commercially available kit: a bottle of R1 was dissolved in 10 mL of R2 to prepare a working solution, 4 µL of serum was added to 200 µL of the working solution in the CK kit and incubated at 37°C for 2 minutes, a wavelength was set to 340 nm under enzyme-linked immunosorbent assay, absorbances A₀, A₁, A₂, and A₃ were read at 0, 1, 2, and 3 minutes respectively, a change in mean absorbance per minute (ΔA) was calculated, and a concentration (U/L) of CK in the serum was calculated.

### Results:

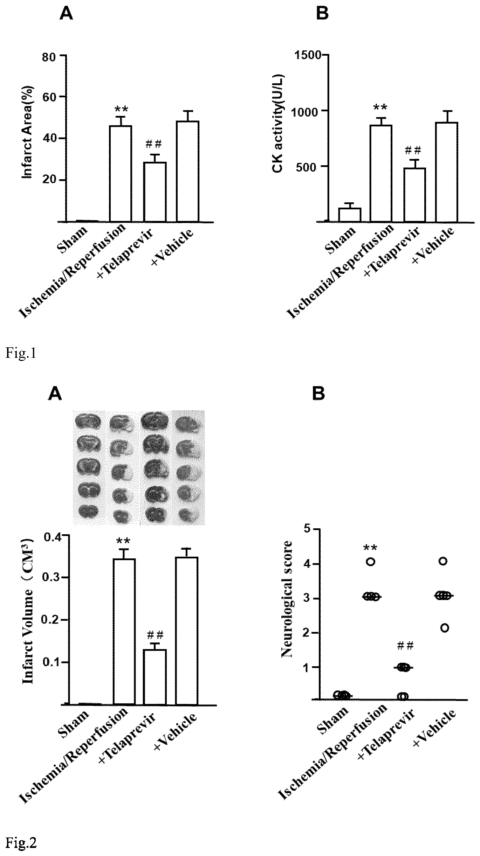

The telaprevir had an anti-myocardial ischemia/reperfusion injury effect in mice, reduced the infarct area of myocardial tissues in mice, reduced the creatine kinase in the serum, reduced myocardial cell death, and had a protective effect on myocardial cells. As shown by A in FIG. 1, administration of telaprevir after 30 minutes of ischemia can significantly reduce the infarct area of myocardial tissues in mice. As shown by B in FIG. 1, administration of telaprevir can significantly reduce the creatine kinase activity of the serum, with data presented as mean ± standard error, n=6-7, **P<0.01 vs Sham, and ^{##}*P*<0.01 vs I/R.

Conclusions: The telaprevir can reduce myocardial cell death, alleviate myocardial ischemia/reperfusion injury, have a protective effect on myocardial cells, and can be used for preparing a drug for alleviating a myocardial ischemia/reperfusion injury to treat myocardial infarction.

### Example 2

Animal experiment: Protective effect of telaprevir on ischemic stroke rat models. Experimental animals: Healthy male SD rats weighing 250-300 g. The experimental animals were raised for one week in an environment with a temperature of 25°C, a relative humidity of 60%, free drinking water, and timed and quantified feeding, and then administered according to the requirements of each experimental group.

Method for building an ischemic stroke rat model: A cerebral ischemia/reperfusion rat model was prepared by a middle cerebral artery occlusion (MCAO) method. The steps were as follows: (1) a common carotid artery (CCA) was isolated, and a left external carotid artery (ECA) and internal carotid artery (ICA) were isolated upwards; (2) the ECA and ICA were temporarily occluded with ophthalmic forceps, and a proximal end of the CCA was ligated; (3) a knotted spare silk suture was placed at a distal end of the CCA, a small incision was cut below the suture, an occlusion suture was inserted into the internal carotid artery, the silk suture was tightened, arterial clips on the ECA and ICA were released, and the occlusion suture was delivered into the cranium along the ICA; (4) when encountering resistance, the insertion depth from the CCA fork was about 18-20 mm; and (5) after 120 minutes of ischemia, the occlusion suture was pulled out, the skin was sewn, and the animal was treated after 24 hours of reperfusion. Success evaluation criteria for the model used Longa "5-score method" to score the neurological impairment of the cerebral ischemia/reperfusion injury rat model. 0 score: no neurological impairment symptoms; 1 score: the right forelimb cannot be fully extended; 2 scores: rightward rotation; 3 scores: walking tilted to the right; 4 scores: unable to walk spontaneously, and loss of consciousness. 1-4 scores represented effective models.

Rat brain TTC staining and infarct volume measurement: After the rat was anesthetized, the brain was quickly taken out, the olfactory bulb and posterior brain were removed, and five coronal brain slices having a thickness of about 2.0 mm were cut from the antiunion, immediately placed in a 1% TTC solution, incubated at 37°C in dark for 30 minutes, and then soaked and immobilized in a 10% paraformaldehyde solution. An area of infarction appeared white, while an area of non-infarction appeared red. Each group of brain slices was arranged neatly and scanned. The infarct area of each brain slice was measured by ImageJ, and an infarct volume of the whole brain was calculated according to a formula: infarct volume = [(sum of front infarct areas of all slices + sum of back infarct areas of all slices)/2] × the thickness of each slice, that is, a sum of infarct volumes of all brain slices.

Experimental groups: the experimental animals were randomly divided into 4 groups, namely:
Sham group: an internal and external carotid artery isolation surgery was performed without inserting an occlusion suture into the artery.
Cerebral ischemia/reperfusion group (I/R group): cerebral ischemia for 2 hours, and reperfusion for 24 hours.
Telaprevir + cerebral ischemia/reperfusion group (Telaprevir + I/R): administered with telaprevir (60 mg/kg) after 2 hours of ischemia and 1 hour of reperfusion.
Vehicle + I/R group (Vehicle + I/R): administered with a vehicle after 2 hours of ischemia and 1 hour of reperfusion.
Neurological function scores of rats were detected and cerebral infarct volumes were measured.

### Results:

The telaprevir had an anti-ischemic/reperfusion injury effect in rats, reduced the infarct volume, improved neurological impairment symptoms, reduced nerve cell death, and had a nerve cytoprotective effect.

As shown by A in FIG. 2, the I/R group had obvious white infarct lesions, the infarct volume of rats administered with telaprevir after 2 hours of ischemia and 1 hour of reperfusion was significantly decreased, and the cerebral ischemia injury was obviously alleviated. As shown by B in FIG. 2, the telaprevir can significantly improve neurological impairment symptoms (data presented as mean ± standard error, n=4-5, ***P*<0.01 vsSham, and ^{##}*P*<0.01 vs I/R).

Conclusions: The telaprevir can reduce nerve cell death, alleviate the cerebral ischemia/reperfusion injury, have a protective effect on nerve cells, and can be used for preparing a drug for alleviating a cerebral ischemia/reperfusion injury to treat ischemic stroke.

### Example 3

Animal experiment: Protective effect of telaprevir on ischemic stroke mouse models. Experimental animals: 7-week-old male C57BL/6J mice. The experimental animals were raised for one week in an environment with a temperature of 25°C, a relative humidity of 60%, free drinking water, and timed and quantified feeding, and then administered according to the requirements of each experimental group.

Method for building an ischemic stroke mouse model: A cerebral ischemia/reperfusion mouse model was prepared by a middle cerebral artery occlusion (MCAO) method. The steps were as follows: after an 8-week-old male C57BL/6J mouse was anesthetized by intraperitoneal injection with 0.3% pentobarbital sodium (20 mL/kg), a left common carotid artery (CCA) was isolated, and a left external carotid artery (ECA) and internal carotid artery (ICA) were isolated upwards; the ECA and ICA were temporarily occluded with ophthalmic forceps, and a proximal end of the CCA was ligated; a knotted spare silk suture was placed at a distal end of the CCA, a small incision was cut below the suture, an occlusion suture was inserted into the internal carotid artery, arterial clips on the ECA and ICA were released, and the occlusion suture was delivered into the cranium along the ICA; and when encountering resistance, the silk suture was tightened, the occlusion suture was secured. After ischemia for 1 hour, the occlusion suture was pulled out, the skin was sewn, and the animal was treated after 24 hours of reperfusion.

Success evaluation criteria for the model used Longa "5-score method" to score the neurological impairment of the cerebral ischemia/reperfusion injury mouse model. 0 score: no neurological impairment symptoms; 1 score: the right forelimb cannot be fully extended; 2 scores: rightward rotation; 3 scores: walking tilted to the right; 4 scores: unable to walk spontaneously, and loss of consciousness. 1-4 scores represented effective models.

Methods for TTC staining in mouse brain tissues and infarct volume measurement were the same as those for TTC staining in rat brain tissues and infarct volume measurement in Example 2.

Experimental groups: the experimental animals were randomly divided into 4 groups, namely:
Sham group: an internal and external carotid artery isolation surgery was performed without inserting an occlusion suture into the artery.
Cerebral ischemia/reperfusion group (I/R group): cerebral ischemia for 1 hour, and reperfusion for 24 hours.
Telaprevir + cerebral ischemia/reperfusion group (Telaprevir + I/R): administered by intramuscular injection with telaprevir (90 mg/kg) (dissolved in 10% DMSO + 30% PEG400 + 60% physiological saline) after 1 hour of ischemia and 1 hour of reperfusion.
Vehicle + cerebral ischemia/reperfusion group (Vehicle + I/R): administered by intramuscular injection with a vehicle (10% DMSO + 30% PEG400 + 60% physiological saline) after 1 hour of ischemia and 1 hour of reperfusion.

Neurological function scores of mice were detected and cerebral infarct volumes were measured.

### Results:

The telaprevir had an anti-ischemic/reperfusion injury effect in mice, reduced the infarct volume, improved neurological impairment symptoms, reduced nerve cell death, and had a nerve cytoprotective effect.

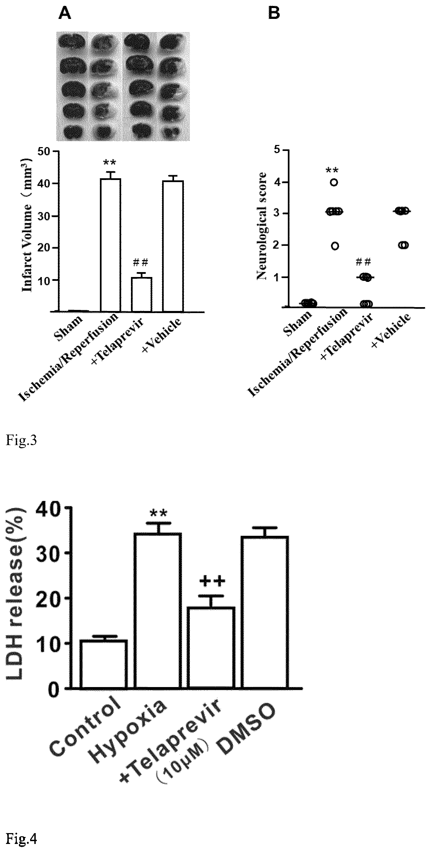

As shown by A in FIG. 3, the I/R group had obvious white infarct lesions, the infarct volume of miceadministered with telaprevir after 1 hourof ischemia and 1 hour of reperfusion was significantly decreased, and the cerebral ischemia injury was obviously alleviated. As shown by B in FIG. 3, the telaprevir can significantly improve neurological impairment symptoms; and data presented as mean ± standard error, n=6, ***P*<0.01 vsSham, and ^{##}*P*<0.01 vs I/R.

Conclusions: The telaprevir can reduce nerve cell death, alleviate the cerebral ischemia/reperfusion injury, have a protective effect on nerve cells, and can be used for preparing a drug for alleviating a cerebral ischemia/reperfusion injury to treat ischemic stroke.

### Example 4

Cell experiment: Protective effect of telaprevir on hypoxic/reoxygenated myocardial cells.

H9c2 myocardial cells were purchased from the Xiangya Cell Bank of Central South University.

**Cell culture method:** Following a conventional cell culture method, the above cells were cultured in a DMEM medium (containing10% of fetal bovine serum). When the cells fused and grew to 80-90%, the cells were digested with a trypsin. After the cells shrank and became round and the intercellular space was obvious, the digestion was immediately terminated with a medium, the cells were dispersed and blown evenly into a single suspension state, passaged in bottles, and cultured in a 37°C cell incubator containing 5% of CO2.

### Building and grouping of hypoxia/reoxygenationinjury models of H9c2 myocardial cells

When the H9c2 cells grew to a degree offusion of 80-90%, the cells were passaged, seeded on plates, and cultured with a normal medium containing 10% FBS DMEM in a 37°C cell incubator containing 95% of air and 5% of CO2. When the cells grew to a degree offusion of 70-80%, the cells were subjected to low serum synchronization treatment for 12 hours, then hypoxia treatment with a serum-free and sugar-free medium in a cell incubator containing 95% of N₂ and 5% of CO₂ for 8 hours, and reoxygenation treatment with a low serum medium in the cell incubator containing 95% of air and 5% of CO₂ for 12 hours.

Specific groups were as follows:
Normal control group (Control group): cultured under normal oxygen conditions;
Hypoxia/reoxygenation group (Hypoxia group): cultured under hypoxia conditions for 8 hours and reoxygenated for 12 hours;
Hypoxia/reoxygenation + telaprevir (Hypoxia + telaprevir 10 µM): during hypoxia/reoxygenation treatment, telaprevir was added to the medium separately, so that the final concentration of the telaprevir in the medium was 10 µM.
Hypoxia/reoxygenation + vehicle group (DMSO): during hypoxia/reoxygenation treatment, a vehicle DMSO in a volume equal to that of telaprevir was added to the medium separately.

Cell injury was detected by an LDH release rate.

### Measurement on lactate dehydrogenase (LDH) release rate

The measurement followed the operating instructions of a commercially available lactate dehydrogenase kit (Shanghai Beyotime Biotechnology Co., Ltd., China). Groups were designed according to experimental requirements, and a background blank control group (without cells) and a maximum enzyme release group were additionally set. An LDH release reagent in a volume of 1/10 of the original medium was added to the maximum enzyme activity well group of the sample, and the culture continued for 1 hour. 120 µL of supernatant was pipetted from each well and added to a new 96-well plate, and 60 µL of LDH detection working solution was added to each well, followed by uniform mixing and incubation at room temperature for 30 minutes (in dark). The absorbance of the sample was measured at 490 nm. Calculation: LDH release rate of cells (%) = (absorbance of the drug treatment group - absorbance of the control group)/(absorbance of the maximum enzyme release group - absorbance of the control group) × 100%. When the absorbance value of each group was calculated, the absorbance value of the background blank control group was first subtracted.

### Experimental results:

The telaprevir had an anti-hypoxia/reoxygenation induced myocardial cell injury effect, reduced hypoxia/reoxygenation induced myocardial cell death, and had a protective effect on myocardial cells.

FIG. 4 is a diagram illustrating effects of telaprevir on the necrosis of H9c2 cells treated by hypoxia/reoxygenation (LDH release rate). It can be seen from the figure that after administration, the telaprevircan significantly reduce the release of LDH in the myocardial cell medium, indicating that the telaprevir had a significant anti-hypoxia/reoxygenation induced myocardial cell injury effect; and data were represented as mean ± standard error, n=3, **P*<0.05, ***P*<0.01 vsControl, ^{#}*P*<0.05, and ^{##}*P*<0.01 vsHypoxia.

The above example further confirmed that the telaprevir can reduce hypoxia/reoxygenation induced myocardial cell death, have a protective effect on myocardial cells, can alleviate the myocardial cell ischemia/reperfusion injury, and can be used for preparing a drug for treating a myocardial cell ischemia/reperfusion injury.

### Example 5

Cell experiment: Protective effect of telaprevir on nerve cells treated by hypoxia/reoxygenation and NMDA.

SH-SY5Y nerve cells were purchased from the Xiangya Cell Bank of Central South University.

**Cell culture method**:Following a conventional cell culture method, the above SH-SY5Y nerve cells were cultured in an RPMI 1640 medium (containing 20% of fetal bovine serum). When the cells fused and grew to 80-90%, the cells were digested with a trypsin. After the cells shrank and became round and the intercellular space was obvious, the digestion was immediately terminated with a medium, the cells were dispersed and blown evenly into a single suspension state, passaged in bottles, and cultured in a 37°C cell incubator containing 5% of CO₂.

### 5.1 Building of hypoxia/reoxygenation injury models of SH-SY5Y nerve cells and drug treatment:

When the SH-SY5Y cells grew to a degree offusion of 80-90%, the cells were passaged, seeded on plates, and cultured with a normal medium containing 20% of FBS RPMI 1640 in a 37°C cell incubator containing 95% of air and 5% of CO₂. When the cells grew to a degree offusion of 70-80%, the cells were subjected to synchronization treatment with a normal medium containing 1% of FBS RPMI 1640 for 12 hours, then hypoxia treatment with a serum-free and sugar-free RPMI 1640 medium in a cell incubator containing 95% of N₂ and 5% of CO₂ for 8 hours (the medium should cover the surface of the cells), and reoxygenation treatment with the normal medium containing 20% FBS RPMI 1640 in the cell incubator containing 95% of air and 5% of CO₂ for 24 hours.

Specific groups were as follows:
Normal control group (Control group): cultured under normal oxygen conditions;
Hypoxia/reoxygenation group (Hypoxia group): treated by hypoxia under sugar-free conditions for 8 hours and reoxygenated for 24 hours;
Hypoxia/reoxygenation group (Telaprevir) (Hypoxia + 1, 5, 10 µMTelaprevir group): telaprevir was added to the medium during hypoxia/reoxygenation, where the telaprevir was first dissolved in DMSO and then diluted to a working concentration, so that the final concentrations of the telaprevir in the corresponding media were 1, 5, and 10 µM, respectively.
Hypoxia/reoxygenation + vehicle group (DMSO): an equal volume of vehicle (DMSO) was added to the medium during hypoxia/reoxygenation.

Cell viability was detected by MTS and cell injury was detected by an LDH release rate.

### 5.2 NMDA induced nerve cell injury models and drug treatment:

The SH-SY5Y cells at the fusion rate of 80-90% or more can be seeded on plates. The cells were inoculated to culture plates with an RPMI-1640 medium containing 20% of fetal bovine serum, cultured in a 37°C cell incubator containing 95% of air and 5% of CO₂, and subjected to synchronization treatment with an RPMI-1640 medium containing 1% of fetal bovine serum for 12 hours when the fusion rate reached 70-80%, then treated with a sugar-free RPMI-1640 medium containing 1 mM NMDA for 30 minutes, and continuously cultured with the RPMI-1640 medium containing 20% of fetal bovine serum for 24 hours. The drug group was administrated with telaprevir.

### Experimental groups:

Normal control group (Control group): cultured with the RPMI-1640 medium without NMDA treatment.
NMDA group: treated with the 1 mM NMDA for 30 minutes and continuously cultured with the RPMI-1640 medium for 24 hours.
NMDA + telaprevir group (Telaprevir): including three concentration groups, namely, telaprevir was added to three NMDA groups, so that the final concentrations of the telaprevir in the media of the three NMDA groups were 1, 5, and 10 µM, respectively, where the telaprevir was first dissolved in DMSO, diluted to a working concentration, and then added to the corresponding medium.
NMDA + vehicle group (DMSO): a vehicle DMSO in a volume equal to that of telaprevirwas addedto the NMDA group.

Cell viability was detected by MTS.

### MTS measurement on cell viability

The measurement followed the operating instructions of a commercially available MTS kit (Promega (Beijing) Biotech Co., Ltd.). Groups were designed according to experimental requirements, and an additional background blank control group (without cells) was set. When the fusion rate of the cells reached 80-90%, the cells were inoculated to 96-well plates. When the fusion rate of the cells reached 70-80%, subsequent experiments were performed. After the experiments, MTS was added in dark to the 96-well plates at a rate of 10 µl/well, and the cells were incubated in dark for about 1 hour. The absorbance of the cells at 490 nm was measured by enzyme-linked immunosorbent assay (the absorbance was controlled at 0.7-1.2), where the absorbance value reflected relative cell viability. Calculation: cell viability (%) = absorbance of the drug group/ absorbance of the control group × 100%. When the absorbance value of each group was calculated, the absorbance value of the background blank control group was first subtracted.

### Measurement on lactate dehydrogenase (LDH) release rate (same as LDH measurement on myocardial cells)

### Experimental results:

The telaprevir had an anti-hypoxia/reoxygenation and NMDA induced SH-SY5Y nerve cell injury, reduced hypoxia/reoxygenation and NMDA induced SH-SY5Y nerve cell death, and had a protective effect on nerve cells.

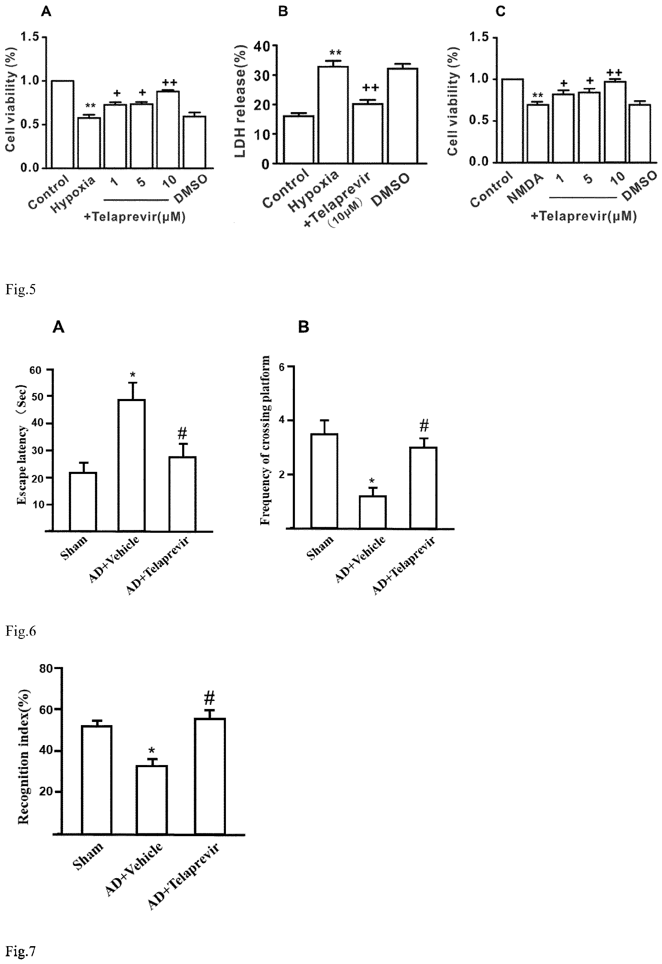

FIG. 5 is a diagram illustrating effects of telaprevir on the viability and necrosis (LDH release rate) of SH-SY5Y cells treated by hypoxia/reoxygenation and NMDA.It can be seen from the figure that after administration, the telaprevir can significantly inhibit a decrease in the viability of hypoxia/reoxygenation (FIG. 5A) and NMDA induced SH-SY5Y nerve cells (FIG. 5C) and an increase in LDH release rate (FIG. 5B), indicating that the telaprevir had an anti-hypoxia/reoxygenation and NMDA induced nerve cell injury effect; and data were represented as mean ± standard error, n=3, *P<0.05, **P<0.01 vsControl, ^{#}*P*<0.05, and ^{##}*P*<0.01 vsHypoxia.

The above example further confirmed that the telaprevir can reduce hypoxia/reoxygenation and NMDA induced nerve cell death, had a protective effect on nerve cells, can alleviate ischemia/reperfusion injury of nerve cells, and can be used for preparing a drug for treating a neuronal ischemia/reperfusion injury.

However, the present invention is not limited to the heart and brain ischemia/reperfusion injury. The liver and kidney ischemia/reperfusion injury has similar mechanisms to the heart and brain ischemia/reperfusion injury, the drug is also suitable for treating a liver and kidney ischemia/reperfusion injury.

### Example 6

Animal experiment: Protective effect of telaprevir on Alzheimer's disease mouse models.

Experimental animals: 7-week-old healthy male ICR mice weighing 25-30 g. The experimental animals were raised for one week in an environment with a temperature of 25°C, a relative humidity of 60%, free drinking water, and timed and quantified feeding, and then administered according to the requirements of each experimental group. Building of an Alzheimer's disease mouse model and drug treatment: Aβ₁₋₄₂ was purchased from MCE Reagent Company, dissolved in DMSO, diluted with physiological saline to 2 µg/ µL, and incubated in an incubator at 37°C for 7 days to convert the Aβ₁₋₄₂ into an aggregated state. The male ICR mouse was anesthetized by intraperitoneal injection with 0.3% pentobarbital sodium (20 mL/kg), the head of the mice was secured with a brain stereotaxic apparatus, and the scalp was cut off with ophthalmic scissors to expose the anterior fontanelle of the mouse. According to a mouse brain stereotaxic map, the hippocampal CA1 (Cornu Amonis1) area of the mouse was located, 2.3 mm behind the anterior fontanelle, 1.8 mm beside the midline, and 2 mm deep. The hippocampus on each side was slowly injected with 2 µL of 2 µg/µL Aβ₁. ₄₂ solution by a micro-injector, and the needle of the injector was retained for 5 minutes and then slowly withdrawn to avoid drug overflow. The Sham group was injected with the same volume of sterile physiological saline. The skull wound was sealed with bone wax, and the incision was sutured. Postoperative intraperitoneal injection of penicillin was performed for 3 days to prevent infection, with a daily injection of 200 mg/kg penicillin.

Experimental groups: the experimental animals were randomly divided into 3 groups, with 6 animals in each group, namely:
Sham group: bilateral hippocampi were injected with an equal amount of physiological saline and injected by intramuscular injection with a vehicle (namely, 10% DMSO + 30% PEG400 + 60% physiological saline for 17 consecutive days) on the 4^{th} day after surgery.
AD + vehicle group: bilateral hippocampi were injected with Aβ₁₋₄₂ and injected by intramuscular injection with a vehicle (namely, 10% DMSO + 30% PEG400 + 60% physiological saline for 17 consecutive days) on the 4^{th} day after surgery.
AD + telaprevir group (Telaprevir): bilateral hippocampi were injected with Aβ₁₋₄₂ and daily injected by intramuscular injection with40 mg/kg of telaprevir (consecutively injected for 17 days, the telaprevir was dissolved in 10% DMSO + 30% PEG400 + 60% physiological saline).

A learning and memory ability and cognitive function of mice were detected by a Morris water maze experiment and a new/old object recognition experiment.

### Detection method:

### (1) Effects of telaprevir on spatial learning and memory ability and cognitive function of Alzheimer's disease model mice - Morris water maze experiment.

According to the requirements of the water maze experiment, a Morris water maze was divided into four quadrants, a platform having a diameter of 12 cm and a height of 35 cm was placed in one of the quadrants, water was added to submerge the platform 1-2 cm, and the pool water was dyed black with carbon ink. After the positioning navigation experiment lasted for 5 days, the platform was removed for a spatial probe experiment, and the time for the mice to reach the original platform position (namely, incubation period) and the number of times they crossed the platform were recorded.

The experimental results were shown in FIG. 6. It can be seen from the figure that, compared with the mice in the Sham group, the mice in the AD + vehicle group showed a significant increase in the time to find the original platform position (incubation period) and a significant decrease in the number of times they crossed the platform, the AD mice after administrated with telaprevir showed a significant decrease in the time to find the original platform position (incubation period) (FIG. 6A) and a significant increase in the number of times they crossed the platform (FIG. 6B) (data presented as mean ± standard error, n=6, **P*<0.05 vs Sham, ^{#}*P*<0.05 vs AD + vehicle group). The results indicated that the telaprevir had a significant improvement effect on the learning and memory ability and cognitive function of Alzheimer's disease mice. (2) Effects of telaprevir on a non-spatial learning and memory ability of Alzheimer's disease model mice - new/old object recognition experiment.

According to the requirements of the new/old object recognition experiment, the experiment included three stages: an adaptation period, a training period, and a testing period. One week before the new object experiment, the experimental mice were touched for 2-3 minutes every day to reduce their tension. 40 × 40 open field test cases were used for the experiment. The experimental mice were placed in a laboratory to adapt to the environment for 20-30 minutes in advance before the experiment. After adapting to the environment, the experimental mice began to receive the new object training experiment. Two objects with identical color, shape, and material (denoted as A and B respectively) were placed in each open field test case, and the mice were trained therein for 10 minutes. A new object testing experiment was performed 24 hours after the training period to evaluate short-term non-spatial learning and memory of the experimental mice. One of the two objects was replaced with another object with a different shape and color (denoted as C), and the time for the experimental mice to probe the two different objects within 10 minutes was recorded. Cognitive index = time spent for probing the new object/ (time spent for probing the new object + time spent for probing the old object) × 100%.

The experimental results were shown in FIG. 7. It can be concluded from the figure that, compared with the mice in the Sham group, the cognitive index of the mice in the AD + vehicle group was significantly decreased, the non-spatial learning and memory ability of the AD mice was weakened, and the cognitive index of the AD mice after administrated with telaprevir was significantly increased (data represented as mean ± standard error, n=6, ***P*<0.01 vs Sham, and ^{##}*P*<0.01 vs vehicle group). The results indicated that the telaprevir had an improvement effect on the non-spatial learning and memory ability of Alzheimer's disease mice.

The above results indicated that the telaprevir in the present invention had a protective effect on nerve cells and a significant improvement effect on the learning and memory ability and cognitive function in the Alzheimer's disease, and can be used in the preparation of a drug for treating or improving the learning, memory, and cognitive function in the Alzheimer's disease, and the indications of the telaprevir were widened. The content illustrated by the above examples should be understood as these examples are merely used for explaining the present invention more clearly, rather than limiting the scope of the present invention. Various equivalent modifications made to the present invention by those skilled in the art after reading the present invention fall within the scope defined by the appended claims of the present application.

## Claims

1. A use of telaprevir in preparation of a drug for treating or preventing an ischemia/reperfusion injury.

2. The use according to claim 1, **characterized in that** the ischemia/reperfusion injury includes at least one of the following: a myocardial ischemia/reperfusion injury, a cerebral ischemia/reperfusion injury, a liver ischemia/reperfusion injury, and a kidney ischemia/reperfusion injury.

3. The use according to claim 2, **characterized in that** the myocardial ischemia/reperfusion injury includes an ischemic heart disease.

4. The use according to claim 2, characterized in thatthe cerebral ischemia/reperfusion injury includes an ischemic stroke.

5. A use of telaprevir in preparation of a cytoprotective drug.

6. The use according to claim 5, **characterized in that** the cytoprotective drug is a drug that has the effects of preventing, inhibiting, or treating injury, degeneration, or dysfunction of tissues, organs, and cells.

7. The use according to claim 5, **characterized in that** the cytoprotective drug is a drug for preventing, inhibiting, or treating cardiovascular diseases, neurological diseases, or ophthalmic diseases; and preferably, the cells include one or more of myocardial cells and nerve cells.

8. The use according to claim 7, **characterized in that** the cardiovascular diseases include one or more of cardiac ischemia and/or vascular ischemia, myocardial infarction, ischemic heart disease, myocardial remodeling, chronic or acute heart failure, hypertrophic cardiomyopathy, cardiac side effects caused by drug therapy, cerebral ischemia/reperfusion injury, liver ischemia/reperfusion injury, and kidney ischemia/reperfusion injury; and the neurological diseases or the ophthalmic diseases include one or more of glaucoma, retinitis pigmentosa, corneal network malnutrition, age-related macular degeneration, wet or dry AMD related photoreceptor degeneration, optic neuropathy, optic neuritis, optic nerve drusen, stroke, Alzheimer's disease, Parkinson's disease, Huntington's disease, Parkinson's plus syndrome, local ischemia, amyotrophic lateral sclerosis, intracranial hemorrhage, cerebral hemorrhage, trigeminal neuralgia, glossopharyngeal neuralgia, myasthenia gravis, muscular atrophy, progressive muscular atrophy, Bell's palsy, progressive bulbar paralysis, spinal muscular atrophy, primary lateral sclerosis, pseudobulbar palsy, invertebrate disc syndrome, cervical spondylosis, hereditary muscle atrophy, plexus disorder, thoracic outlet disruption syndrome, porphyria, peripheral neuropathy, multiple system atrophy, cortical basal ganglia degeneration, progressive supranuclear palsy, dementia with lewy bodies, demyelinating disease, frontotemporal lobe dementia, Guillain-Barre syndrome, multiple sclerosis, Creutzfeldt-Jakob disease, Charcot-Marie-Tooth disease, prion disease, fatal familial insomnia, Gerstmann-Straussler-Scheinker syndrome, bovine spongiform encephalopathy, epilepsy, Pick's disease, AIDS dementia syndrome, nerve injury caused by exposure to toxic compounds in a group consisting of industrial solvents, heavy metals, drugs, and chemotherapy agents, and nervous system injury caused by mechanical, physical, or chemical trauma.

9. The use according to claim 5, **characterized in that** the organs include one or more of the brain, lungs, heart, blood vessels, kidneys, pancreas, skin, eyes, and corneas.

10. The use according to claim 8, **characterized in that** the neurological diseases include an Alzheimer's disease.

11. A use of telaprevir in treatment or prevention of an ischemia/reperfusion injury.

12. The use according to claim 11, **characterized in that** the ischemia/reperfusion injury includes one or more of a myocardial ischemia/reperfusion injury, a cerebral ischemia/reperfusion injury, a liver ischemia/reperfusion injury, and a kidney ischemia/reperfusion injury.

13. The use according to claim 12, **characterized in that** the myocardial ischemia/reperfusion injury includes an ischemic heart disease.

14. The use according to claim 12, **characterized in that** the cerebral ischemia/reperfusion injury includes an ischemic stroke.

15. A method for treating or preventing an ischemia/reperfusion injury with telaprevir, **characterized in that** an effective dose of telaprevir is administrated to patients or in vitro tissues and organs.

16. The method according to claim 15, **characterized in that** the ischemia/reperfusion injury includes one or more of a myocardial ischemia/reperfusion injury, a cerebral ischemia/reperfusion injury, a liver ischemia/reperfusion injury, and a kidney ischemia/reperfusion injury.

17. The method according to claim 15, **characterized in that** the myocardial ischemia/reperfusion injury includes an ischemic heart disease.

18. The method according to claim 15, **characterized in that** the cerebral ischemia/reperfusion injury includes an ischemic stroke.

19. A use of telaprevir in protection of cells.

20. The use according to claim 19, **characterized in that** the cells include one or more of myocardial cells and nerve cells.

21. A method for protecting cells with telaprevir, **characterized in that** an effective dose of telaprevir is administrated to patients or in vitro tissues and organs.

22. The method according to claim 21, **characterized in that** the cells include one or more of myocardial cells and nerve cells.

23. A use of telaprevir in treatment or prevention of an Alzheimer's disease.

24. A method for treating or preventing an Alzheimer's disease with telaprevir, **characterized in that** an effective dose of telaprevir is administrated to patients.
